# EUROPÄISCHE PATENTSCHRIFT

(11) **EP 1 992 592 B1**
(45) Veröffentlichungstag und Bekanntmachung des Hinweises auf die Patenterteilung: **12.10.2011**
(21) Anmeldenummer: 08007345.5
(22) Anmeldetag: 15.04.2008
(51) Int. Cl.: C01B 7/04, C07C 263/10

(54) **Verfahren zur Oxidation eines Chlorwasserstoff enthaltenden Gasgemisches**
Method for oxidising a gas mixture containing hydrogen chloride
Procédé d'oxydation d'un mélange de gaz contenant du chlorure d'hydrogène

(30) Priorität: 27.04.2007 DE 102007020444
(43) Veröffentlichungstag der Anmeldung: 19.11.2008
(73) Patentinhaber: Bayer MaterialScience AG, 51368 Leverkusen (DE)
(72) Erfinder: Haas, Michel, Dr.Dr., 41539 Dormagen (DE); Bruns, Rainer, Dr., 51373 Leverkusen (DE); Loddenkemper, Tim, 41542 Dormagen (DE)

(56) Entgegenhaltungen:
- WO-A-2004/037718
- WO-A-2007/066810
- JP-A- 2004 345 883
- JP-A- 2004 345 884
- JP-A- 2005 177 614
- US-A1- 2004 141 901
- US-A1- 2005 025 693

## Beschreibung

Die vorliegende Erfindung betrifft ein Verfahren zur Herstellung von Chlor aus einem Chlorwasserstoff sowie weitere Nebenkomponenten wie Schwefelverbindungen enthaltenden Gasgemisch, dadurch gekennzeichnet, dass der eingesetzte Chlorwasserstoff weniger als 100ppm, bevorzugt weniger als 50ppm, besonders bevorzugt weniger als 5ppm, ganz besonders bevorzugt weniger als 1ppm, an Schwefel in elementarer oder gebundener Form, bezogen auf das Gewicht des Gasgemisches, enthält.

Eine Vielzahl von chemischen Verfahren zur Umsetzung mit Chlor oder Phosgen, wie die Herstellung von Isocyanaten oder Chlorierungen von Aromaten, führen zu einem Zwangsanfall an Chlorwasserstoff. In der Regel wird dieser Chlorwasserstoff durch Elektrolyse wieder in Chlor umgewandelt (Vgl. z.B. WO 97 24320 A1). Gegenüber dieser sehr energieaufwändigen Methode, bietet die thermische Oxidation von Chlorwasserstoff mit reinem Sauerstoff oder einem sauerstoffhaltigen Gas an heterogenen Katalysatoren (den sogenannten Deacon-Prozess) gemäß

4HCl + O₂ ⇔ 2Cl₂ + 2H₂O

deutliche Vorteile hinsichtlich des Energieverbrauchs (s. z.B. WO-A- 04/0 14 845).

Die katalytische Oxidation von HCl-Gas mit O₂ zu Cl₂ und H₂O wird typischerweise an heterogenen Katalysatoren durchgeführt. Man bedient sich verschiedenster Katalysatoren, z.B. auf Basis von Ruthenium, Chrom, Kupfer, etc, geträgert oder ungeträgert. Solche Katalysatoren sind beispielsweise in JP 2001 019405, DE 1 567 788 A1, EP 251 731 A2, EP 936 184 A2, EP 761 593 A1, EP 711 599 A1 und DE 105 50 131 A1 beschrieben. Insbesondere Katalysatoren auf Basis von metallischem Ruthenium, Rutheniumoxid, Rutheniummischoxid, Rutheniumoxichlorid und Rutheniumchlorid, geträgert oder ungeträgert, können hier verwendet werden. Geeignete Träger sind in diesem Zusammenhang z.B. Zinnoxid, Aluminiumoxid, Siliziumoxid, Aluminium-Silizium Mischoxide, Zeolite, Oxide und Mischoxide (z.B. von Titan, Zirkon, Vanadium, Aluminium, Silizium, etc.), Metallsulfate, Ton. Die Auswahl der möglichen Träger ist allerdings nicht auf diese Auflistung beschränkt.

Es wurde nun gefunden, dass Schwefelkomponenten, wie z.B. H₂SO₄, SO₂, SO₃, H₂S oder COS als Katalysatorgifte wirken. Diese Schwefelkomponenten setzen sich langsam über der gesamten Katalysatorschüttung nach und nach ab. Hierdurch reduziert sich die katalytische Aktivität, was für eine großtechnische Anwendung nicht vertretbar ist. Der Aktivitätsverlust des Katalysators kann dauerhaft oder temporär, reversibel oder irreversibel sein. Ein weiterer Grund des Aktivitätsverlustes liegt an der Tatsache, dass die meisten Deacon- Katalysatoren thiophil sind und deshalb auch unter sehr sauberen Bedingungen mehr oder weniger stabile Verbindungen mit Schwefel bilden und somit die katalytisch aktiven Komponenten unzugänglich machen oder deaktivieren. Für ein optimales Betreiben des Deacon-Verfahrens ist demnach ein möglichst geringer Gehalt an Schwefelkomponenten im HCl-Gas notwendig.

Bei den meisten Prozessen wie der Herstellung von Isocyanaten durch Phosgenierung können aber erhebliche Mengen an Schwefelkomponenten als Verunreinigung im HCl-Abgas enthalten sein und in den Deacon-Prozess eingeführt werden. Die Schwefelkomponenten können ihren Ursprung im Erdgas/Kohle haben, die für die Herstellung von Phosgen eingesetzt wird, d.h. ein Eintrag der Schwefelkomponenten über die CO Qualität in die Phosgenherstellung und nachfolgend über das HCl Prozessgas der Isocyanatanlage in den Deacon-Prozess kann stattfinden. Weitere Schwefelquellen können im Isocyanat-Prozess vorhanden sein und den HCl-Gasstrom zur HCl-Oxidation belasten. Ihren Ursprung könnten diese Schwefelquellen in im Isocyanatverfahren verwendeten Zuschlagsstoffen, z.B. in der Destillation, in der Qualität der verwendeten Katalysatoren, z.B. für die Herstellung und Vernichtung von Phosgen, als auch in der Reinheit der verwendeten Lösungsmittel haben.

Da schon geringste Mengen an Schwefel den Deacon-Katalysator reversibel oder irreversibel schädigen können, ist hier eine aufwendige und umfassende Reinigung des Gases vorzunehmen bevor das Isocyanat-Prozessgas in Kontakt mit dem Deacon Katalysator gerät. Diese Reinigung der Edukte bevor sie in den Deacon-Reaktor gelangen, ist daher essentiell für die Katalysator-Lebensdauer und demzufolge für die Wirtschaftlichkeit der Chlorherstellung aus HCl durch katalytische Oxidation.

In JP 2005-177614 wird die Entfernung von Schwefelkomponenten aus HCl- bzw. Cl2-haltigem Gas beschrieben. Die Entfernung geschieht durch Kontakt dieser Gase mit Metallen oder deren Verbindungen. Die Metalle werden aus den Gruppen 8 - 10 des Periodensystems der Elemente ausgewählt.

Der vorliegenden Erfindung liegt daher die Aufgabe zugrunde, ein möglichst effizientes Verfahren zur Reduzierung von Schwefelkomponenten im HCl-haltigen Prozess-Gas aus einer Isocyanatanlage, das anschließend insbesondere einem Deacon- oder Deacon-ähnlichen Verfahren zur Oxidation des Chlorwasserstoffs mit Sauerstoff zugeführt werden soll, bereitzustellen.

Eine weitere Aufgabe der vorliegenden Erfindung ist es, ein HCl-haltiges Prozessgas aus einer Isocyanatanlage mit einem geringen Gehalt an Schwefelkomponenten im HCl-haltigen Prozessgas für ein sich anschließendes Deacon oder Deacon-ähnliches Verfahren zur Oxidation des Chlorwasserstoffes mit Sauerstoff zur Verfügung zu stellen.

Das Chlorwasserstoff und Schwefel enthaltende Gas, das in dem erfindungsgemäßen Verfahren eingesetzt wird, ist im Allgemeinen das Prozessgas einer Phosgenierungsreaktion zur Bildung organischer Isocyanate, alternativ kann es sich aber auch um Prozessgase von Chlorierungsreaktionen von Kohlenwasserstoffe handeln.

Die vorliegende Erfindung betrifft auch ein Verfahren zur Herstellung von Isocyanaten durch Phosgenierung der entsprechenden Amine, in dem
a) Kohlenmonoxid mit Chlor zu Phosgen umgesetzt wird, und
b) die Amine mit Phosgen zu den Isocyanaten umgesetzt werden, wobei ein rohes Isocyanatgemisch und ein Gasstrom enthaltend Chlorwasserstoff erhalten wird, und
c) das rohe Isocyanatgemisch destillativ gereinigt wird, wobei das Isocyanat und ein Gemisch enthaltend Isocyanat und nicht verdampfbaren Rückstand in Gehalten von 10 bis 70 Gew.% nicht verdampfbarer Isocyanat-Rückstand, bezogen auf das Gewicht des Gemisches erhalten wird, und
d) das in dem Schritt c) erhaltene Gemisch aus Isocyanat und nicht verdampfbarem Rückstand in Gehalten von 10 bis 70 Gew.-% einem weiteren Aufarbeitungsschritt zugeführt wird, bei dem Isocyanat durch Verdampfung in einem Knetertrockner oder in Rückstandspfannen zurück gewonnen wird oder das Amin durch Hydrolyse des Rückstands zurück gewonnen wird und, das zurückgewonnene Isocyanat oder das zurückgewonnene Amin zumindest teilweise in die Umsetzung in Schritt b) zurückgeführt wird, und
e) aus dem in Schritt b) erhaltenen Gasstrom enthaltend Chlorwasserstoff durch katalytische Oxidation Chlor hergestellt wird, wobei der Gehalt an Schwefel in elementarer oder gebundener Form in dem in der katalytischen Oxidation eingesetzte Gasstrom enthaltend Chlorwasserstoff insgesamt weniger als 100ppm, bevorzugt weniger als 50ppm, besonders bevorzugt weniger als 5ppm, ganz besonders bevorzugt weniger als 1ppm, bezogen auf das Gewicht des eingesetzten Gasstroms, beträgt, und
f) das in Schritt e) hergestellte Chlor zumindest in Teilen in die Herstellung von Phosgen in Schritt a) zurückgeführt wird.

Dabei ist die Konzentrationsangabe in ppm auf das Gewicht bezogen.

Das erfindungsgemäß umgesetzte Chlorwasserstoff-Gas, das Schwefelverbindungen enthält, kann weitere Bestandteile, wie insbesondere Kohlenwasserstoffe, CO, CO₂, Stickstoffkomponenten, etc. umfassen.

Schwefel in elementarer Form ist Schwefel, der in Form von ausschließlich Schwefel enthaltenden Molekülen vorliegt. Unter Schwefel in gebundener Form werden Verbindungen oder Moleküle verstanden, die neben Schwefel noch weitere, von Schwefel verschiedene Atome enthalten wie beispielsweise Schwefelwasserstoff, COS und Schwefelsalze. Unter Gesamt-Schwefel wird Schwefel und Schwefel in gebundener Form verstanden.

Die analytische Bestimmung des Schwefelgehalts kann chromatographisch, insbesondere gaschromatographisch, und bevorzugt in Kopplung mit Massenspektrometrie erfolgen. Alternativ dazu kann auch eine Anreicherung der Schwefelkomponenten mittels eines Adsorbers erfolgen sowie anschließende Untersuchung des Adsorbers auf Schwefel.

Die Herstellung von Phosgen aus Kohlenmonoxid und Chlor in Schritt a) ist an sich bekannt, beispielsweise aus EP-B-881 986 oder EP-A-1640341. Die Umsetzung des Kohlenmonoxids erfolgt durch Umsetzung des Kohlenmonoxids mit Chlor zu Phosgen, zum Beispiel, an einem Aktivkohle-Katalysator. Alternativkatalysatoren können aber auch eingesetzt werden. Hier kann auf den Stand der Technik verwiesen werden (z.B. DE 3327274; GB 583477; WO 97/30932; WO 96/16898; US 6713035) Im technischen Maßstab wird Phosgen überwiegend durch Umsetzung von Kohlenmonoxid mit Chlor bevorzugt an Aktivkohle als Katalysator hergestellt. Die stark exotherme Gasphasenreaktion erfolgt bei Temperaturen von mindestens 100°C bis maximal 600°C in der Regel in Rohrbündelreaktoren. Die Abführung der Reaktionswärme kann auf unterschiedliche Weise erfolgen, beispielsweise durch ein flüssiges Wärmetauschmittel, wie z.B. in WO 03/072237 beschrieben, oder durch Siedekühlung über einen Sekundärkreislauf unter gleichzeitiger Nutzung der Reaktionswärme zur Dampferzeugung, wie z.B. in US4764308 offenbart. Bevorzugt wird im Schritt a) Aktivkohle als Katalysator eingesetzt, wobei die Aktivkohle einen Gehalt an Gesamtschwefel von kleiner 1 Gew-%, insbesondere kleiner 0.5 Gew.-% bezogen auf das Gesamtgewicht des Katalysators enthält. Weiterhin wird der erfindungsgemäße Schritt a) bevorzugt bei Temperaturen kleiner gleich 300 °C durchgeführt und weiterhin wird der erfindungsgemäße Schritt a) in Kombination mit der Erzeugung von Dampf kombiniert. Weiterhin bevorzugt hat der Katalysator nach Schritt a) eine spezifische Oberfläche von größer 10 m2/g.

Die Verwendung des bevorzugten Katalysators zur Herstellung von Phosgen unter den bevorzugten Bedingungen in Schritt a) führt zu einem deutlich verringerter Eintrag von Schwefelkomponenten in das Chlorwasserstoffhaltige Prozessgas von Schritt b), das in einer katalytischen Oxidationsreaktion in Schritt e) zu Chlor umgesetzt wird und ermöglicht damit eine deutlich verlängerte Lebenszeit des in Schritt e) eingesetzten Katalysators.

Die Herstellung von Isocyanaten durch Phosgenierung von primären Aminen und die anschließende destillative Aufreinigung des rohen Isocyanates sind allgemein bekannt. Für das erfindungsgemäße Verfahren nach Schritt b) können primäre Amine verwendet werden. Besonders geeignet sind Amine, insbesondere Diamine, auf Basis von aliphatischen oder cycloaliphatischen Kohlenwasserstoffen mit 1 bis 15 Kohlenstoffatomen. Besonders gut geeignete Amine sind 1,6-Diamino-hexan (HDA), 1-Amino-3,3,5-trimethyl-5-aminomethylcyclohexan (IPDA) und 4,4'-Diaminodicyclohexylamin. Bevorzugt verwendet wird 1,6-Diamino-hexan. Ebenfalls können für das erfindungsgemäße Verfahren aromatische Amine verwendet werden. Beispiele für bevorzugte aromatische Amine sind Toluylendiamin (TDA), insbesondere 2,4-TDA und 2,6-TDA und Gemische daraus, Diaminobenzol, Naphtyldiamin (NDA) und 2,2'-2,4' oder 4,4'-Methylendiphenyldiamin (MDA) oder Isomerengemische davon. Besonders bevorzugt ist Toluylendiamin (TDA), insbesondere 2,4-TDA und 2,6-TDA und Gemische daraus. Ebenso besonders bevorzugt ist 2,2'-2,4' oder 4,4'-Methylendiphenyldiamin (MDA) und Gemische daraus.

Die Umsetzung von Aminen mit Phosgen ist grundsätzlich bekannt und zum Beispiel beschrieben in Ullman's Encyclopedia of Industrial Chemistry, 5th ed. Vol. A 19 p. 390ff, VCH Verlagsgesellschaft mbH, Weinheim, 1991 und G. Oertel (Ed.) Polyurethane Handbook, 2nd Edition, Hanser Verlag, München, 1993, p.60ff, sowie G. Wegener et. Al., Applied Catalysts A: General 221 (2001), p.303-335, Elsevier Science B.V.

Bevorzugt findet die Umsetzung von beispielsweise TDA und Phosgen in Schritt b) wie folgt statt: Aus TDA wird durch Umsetzung mit Phosgen in Verfahrensschritt b) TDI hergestellt. Bevorzugt stammt das TDA aus der Hydrierung von Dinitrotoluol (DNT). Der Verfahrensschritt b) wird nachfolgend auch als Phosgenierung bezeichnet. Die Umsetzung erfolgt unter Bildung von Chlorwasserstoff als Nebenprodukt.

Die Synthese von Isocyanaten im Allgemeinen und von TDI im Besonderen ist aus dem Stand der Technik hinlänglich bekannt, wobei in der Regel Phosgen in einem stöchiometrischen Überschuss, bezogen auf Amin, bevorzugt TDA, eingesetzt wird. Üblicherweise findet die Phosgenierung in Schritt b) in der Flüssigphase statt (DE 3744001 C1, EP 0314985 A1), wobei das Phosgen und TDA in einem Lösemittel gelöst sein können. Bevorzugte Lösemittel sind chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α-bzw. β-Naphthylchlorid, Benzoesäureethylester, Phthalsäuredialkylester, Diisodiethylphthalat, Toluol und Xylole. Weitere Beispiele für geeignete Lösemittel sind aus dem Stand der Technik bekannt. Wie außerdem aus dem Stand der Technik, z.B. WO-A-96/16028, bekannt, kann als Lösemittel für Phosgen ebenso das gebildete Isocyanat selbst fungieren. In einer anderen, bevorzugten Ausführungsform findet die Phosgenierung oberhalb des Siedepunktes des TDA, statt. Die Gasphasenphosgenierung ist z.B. in EP 570 799 A, EP 1555258 A1, EP 1526129 A1 oder DE 10161384 A1 beschrieben. Vorteile dieses Verfahrens gegenüber der ansonsten üblichen Flüssigphasenphosgenierung liegen in der Energieeinsparung, bedingt durch die Minimierung eines aufwändigen Lösemittel- und Phosgenkreislaufs.

Das TDA kann mit Phosgen in einer einstufigen oder zweistufigen oder ggf. mehrstufigen Reaktion umgesetzt werden. Dabei ist eine kontinuierliche wie auch diskontinuierliche Betriebsweise möglich.

Wird eine einstufige Phosgenierung in der Gasphase gewählt, so erfolgt die Umsetzung oberhalb der Siedetemperatur des TDA bevorzugt innerhalb einer mittleren Kontaktzeit von 0,05 bis 5 Sekunden und bei Temperaturen von 200 bis 600°C (DE 10161384 A1). Wird die Phosgenierung in der Gasphase gewählt, so erfolgt diese Umsetzung bevorzugt adiabat. Weiterhin bevorzugt wird die Phosgenierung bevorzugt in der Gasphase in einem Rohrreaktor ohne bewegliche Einbauten durchgeführt, wobei die Gase in dem Reaktor bevorzugt rückvermischungsfrei geführt werden.

Bei der Phosgenierung in der Flüssigphase werden üblicherweise Temperaturen von 20 bis 240°C und Drücke von 1 bis ca 50 bar eingesetzt (US-A-3,544,611). Die Phosgenierung in der Flüssigphase kann einstufig oder mehrstufig durchgeführt werden, wobei Phosgen im stöchiometrischen Überschuss eingesetzt werden kann. Dabei werden die TDA-Lösung und die Phosgenlösung bevorzugt über ein statisches Mischelement vereinigt und anschließend beispielsweise von unten nach oben durch einen oder mehrere Reaktionstürme geführt, wo das Gemisch zum gewünschten Isocyanat ausreagiert. Neben Reaktionstürmen, die mit geeigneten Mischelementen versehen sind, können auch Reaktionsbehälter mit Rührvorrichtung eingesetzt werden. Außer statischen Mischelementen können auch spezielle dynamische Mischelemente Anwendung finden. Geeignete statische und dynamische Mischelemente sind aus dem Stand der Technik bekannt.

In der Regel wird die kontinuierliche Flüssigphasen-Isocyanatherstellung im industriellen Maßstab zweistufig durchgeführt. Dabei wird in der ersten Stufe im Allgemeinen bei Temperaturen von maximal 220°C, bevorzugt maximal 160°C aus Amin und Phosgen das Carbamoylchlorid sowie aus Amin und abgespaltenem Chlorwasserstoff Aminhydrochlorid gebildet. Diese erste Stufe ist stark exotherm. In der zweiten Stufe wird sowohl das Carbamoylchlorid zu TDI und Chlorwasserstoff gespalten als auch das Aminhydrochlorid zum Carbamoylchlorid umgesetzt. Die zweite Stufe wird in der Regel bei Temperaturen von mindestens 90°C, vorzugsweise von 100 bis 240°C, durchgeführt.

Bevorzugt erfolgt die Phosgenierung in der Gasphase, da die Gasphasenphosgenierung im Allgemeinen bei einem höheren Druck als die Flüssigphasenphosgenierung durchgeführt wird und eine anschließende Verdichtung von HCl vor Eintritt in die katalytische Oxidation somit vereinfacht wird. Bevorzugt liegt der absolute Druck in der Phosgenierung daher im Bereich von 1 bis 30 bar, bevorzugt von 1 bis 6 bar.

Bevorzugt erfolgt die Phosgenierung in der Gasphase, da die in der Gasphasenphosgenierung zugesetzten Inerten, insbesondere Stickstoff, zu einer Verdünnung des Chlorwasserstoffhaltigen Prozessgasstromes zur katalystischen Oxidation zu Chlor nach Schritt e) führen, wodurch die Exothermie der katalytischen Oxidation von HCl zu Chlor besser und leichter kontrolliert und die Abfuhr der Reaktionswärme in Schritt e) vereinfacht wird. Der Zusatz der Inerten in der Gasphasenphosgenierung verbessert die Wärmeabfuhr in Schritt e) und verhindert dadurch lokale Überhitzung des Katalysators, d.h. die Lebensdauer des Katalysators wird somit verlängert.

Über die in der Phosgenierung in der Flüssigenphase oder Gasphase eingesetzten Lösungsmittel werden im Lösemittel enthaltende Schwefelkomponenten in deren Chlorwasserstoffhaltigen Prozessgasstrom aus Schritt b) in die katalytische HCl Oxidation zu Chlor nach Schritt e) eingetragen. Insbesondere bevorzugt wird für die Phosgenierung ein Lösungsmittel mit einem Gehalt an Gesamtschwefel von kleiner 5ppm, bevorzugt von kleiner 3ppm eingesetzt.

Bevorzugt wird die Phosgenierung in der Gasphase durchgeführt, da der gesamte Lösemittel Hold-up (die gesamte Lösemittel-Menge) bei diesem Verfahren deutlich gegenüber der Phosgenierung in der flüssigen Phase verringert ist und somit auch der Hold-up an Gesamt-Schwefel deutlich verringert ist. Dieses führt zu einer deutlich verlängerten Standzeit des Katalysators in Schritt e).

Bevorzugt wird die Phosgenierung in Schritt b) in der Gasphase durchgeführt, da die Schrift EP0570799 lehrt, dass die Ausbeuten bei der Phosgenierung in der Gasphase oberhalb von 95% liegen. Die hohe Ausbeute führt zu geringerem Anfall von nicht verdampfbaren TDI Rückstandsgemisch, das in Schritt d) aufgearbeitet werden muss. Damit sinkt die ggf. in Schritt d) einzusetzende Menge ggf. Schwefelhaltiger Zuschlagsstoffe und dieses führt zu einem geringeren Gehalt an Schwefel in dem Chlorwasserstoffhaltigen Prozessgas, das in Schritt e) katalytisch zu Chlor oxidiert wird. Somit wird durch die Phosgenierung in der Gasphase in Schritt b) die Lebenszeit des Katalysators in Schritt e) verlängert.

Der flüssige Produktstrom, das rohe Toluylendiisocyanat, wird in Schritt c) anschließend einer destillativen, im Allgemeinen mehrstufigen Aufarbeitung zugeführt, wobei im rohen Isocyanatgemisch aus Schritt b) noch gelöstes Phosgen sowie das Lösemittel abgetrennt, zurück gewonnen und dem Prozess wieder zugeführt werden. Die Destillation des rohes Isocyanatgemisches in Schritt c) kann nach allgemein bekannten Methoden (wie zum Beispiel in EP-A-1413571 und US 2003/0230476 A1 beschrieben). Die Destillation erfolgt im allgemeinen mehrstufig und ist beispielsweise in WO-A-2004/056757 und EP-B-1575907 beschrieben.

All den bekannten Verfahren zur destillativen Reinigung des rohen TDI in Schritt c) ist jedoch gemeinsam, dass neben dem gewünschten gereinigten TDI aus der Destillation ein Gemisch enthaltend Toluylendiisocyanat und Destillationsrückstand erhalten wird, das weiterbehandelt werden muss. Die Abtrennung des TDI-Rückstandgemisches, das in Schritt d) weiterer bearbeitet wird, kann dabei zu Beginn, in der Mitte und am Ende der Destillationssequenz aus den Sumpfströmen der jeweiligen Kolonnen erfolgen.

Die Wiedergewinnung des Lösemittels in der Destillation in Schritt c) und / oder in der weiteren Aufarbeitung in Schritt d) und Rückführung in den Phosgenierungschritt b) führt dazu, dass im Lösemittel vorhandene Schwefelkomponenten in das Chlorwasserstoffhaltige Prozessgas aus Schritt b), den Gasstrom enthaltend Chlorwasserstoff, gelangen und in der Konsequenz den Katalysator in Schritt e) schädigen können. Weiterhin führt die Rückführung von Lösemittel aus der Destillation in Schritt c) und / oder aus der weiteren Aufarbeitung in Schritt d) dazu, dass Schwefelkomponenten aus im Schritt c) und / oder d) eingesetzten Zuschlagsstoffen über den Lösemittelkreislauf in den Phosgengenierungsschritt b) und damit in das Chlorwasserstofflialtige Prozessgas, das in Schritt e) katalytisch oxidiert wird, eingetragen wird. Somit können Schwefelkomponenten in den in Schritt c) und / oder d) verwendeten Zuschlagsstoffen zu einer Verringerung der Lebensdauer des Katalysators in Schritt e) führen.

In Schritt d) wird aus dem in Schritt c) erhaltene Gemisch enthaltend Isocyanat und nicht verdampfbarem TDI-Destillationsrückstand mit einem Gehalt an Rückstand von 1 bis 70 Gew.-%, bevorzugt von 20 bis 70 Gew.-%, bezogen auf das Gewicht des Gemisches, einem weiteren Aufarbeitungsschritt unterworfen, um eine möglichst hohe TDI-Ausbeute zu erzielen und der Anfall zu entsorgender Reststoffe zu minimieren.

Hierzu kann das Gemisch enthaltend Isocyanat und nicht verdampfbarem TDI Rückstand zunächst über einen Verdampfer weiter aufkonzentriert werden, wobei weiteres Isocyanat gewonnen und der Destillation in Schritt c) zugeführt wird und ein Gemisch enthaltend Isocyanat und nicht verdampfbarer Isocyanat-Rückstand in Gehalten von größer 30 Gew.-% nicht verdampfbarer Isocyanat-Rückstand, bevorzugt größer 40 Gew.-% nicht verdampfbarer Isocyanatrückstand bezogen auf das Gewicht des Gemisches erhalten wird.

### Dieses Gemisch kann nach mehreren möglichen Verfahren aufgearbeitet werden:

In einer ersten Ausführungsform erfolgt die Aufarbeitung in einer Rückstandspfanne, d.h. in einem gerührten und beheizten Behälter, wobei hochsiedende Kohlenwasserstoffe (vorzugsweise Bitumen), die unter den Destillationsbedingungen inert sind, eingemischt werden, um möglichst vollständig das im Rückstand noch vorhandene freie Isocyanat abzudestillieren. Der verbleibende Rückstand kann als rieselfähiger Feststoff ausgetragen werden und einer Verbrennung zugeführt werden wie in EP 0548685 A2 beschrieben. Nachteilig ist hierbei, dass das Bitumen erhebliche Mengen an Schwefelverbindungen enthalten kann. Diese Schwefelverbindungen können unter den Destillationsbedingungen ausgetragen werden und werden dann in den nachfolgenden Wäschern mit Lösemittel aus dem Destillationsgasstrom ausgewaschen. Das mit den Schwefelverbindungen kontaminierte Lösemittel wird wie oben beschrieben in der Phosgenierung nach Schritt b) eingesetzt und führt damit zu einer Verringerung der Lebenszeit des Katalysators in Schritt e). Bevorzugt wird in dieser Ausführungsform Bitumen mit Gesamt-Schwefelgehalten kleiner 5-Gew.%, bevorzugt nahezu schwefelfreies Bitumen mit Gesamt-Schwefelgehalten von kleiner 1-Gew.% eingesetzt.

In einer alternativen Ausführungsform erfolgt die Aufarbeitung in Schritt d) durch den Einsatz von Knetertrocknern (US 5,446,196). In diesem Verfahren werden die oben beschriebenen beheizten und gerührten Behälter durch Knetertrockner ersetzt. Durch Einsatz von zum Beispiel Bitumen wird wie im oben genannten Beispiel der verbleibende Rückstand als rieselfähiger Feststoff erhalten der als Brennstoff zum Beispiel in Zementwerken eingesetzt werden kann. Bevorzugt wird auch in dieser Ausführungsform Bitumen mit Gesamt-Schwefelgehalten kleiner 5-Gew.%, bevorzugt nahezu schwefelfreies Bitumen mit Gesamt-Schwefelgehalten von kleiner 1-Gew.% eingesetzt.

In einer weiteren bevorzugten Ausführungsform erfolgt die Aufarbeitung in Schritt d) durch den Einsatz von Knetertrockern mit mindestens zwei rotierenden Wellen, wodurch auf den Einsatz von Bitumen verzichtet werden kann. Geeignete Knetertrockner mit zwei rotierenden Wellen sind beispielsweise in DE 10120391 A1 offenbart.

In einer weiteren besonders bevorzugten Ausführungsform erfolgt die Aufarbeitung nach Schritt d) in einer Druckhydrolyse mit Wasser, wobei die in Schritt b) eingesetzten primären Amine erhalten werden. Die Druckhydrolyse ist an sich bekannt und beispielsweise in WO-A-2007/007887 oder WO-A-2007/007791 oder US-B-6,673,960 beschrieben. Die Druckhydrolyse ist insbesondere bevorzugt, wenn es sich bei dem Isocyanat um TDI handelt. In einer weiteren bevorzugten Ausführungsform erfolgt die Hydrolyse unter Verwendung von nahezu schwefelfreien Zuschlagsstoffen mit einem Gesamt-Schwefelgehalt kleiner 1 Gew.%.

Im Rückstand befinden sich nicht unerhebliche Mengen an Verbindungen, aus welchen TDA über eine Hydrolyse zurückgewonnen werden kann wie z.B. Carbodiimide, Harnstoffe, Uretdione etc. Daher kann anstelle der Knetertrockner oder Rückstandspfannen, mit denen schwefelhaltiges Bitumen eingesetzt werden muss, bevorzugt in Schritt d) zunächst Isocyanat und ggf. Chlorwasserstoff und Lösungsmittel in einem Verdampfer abgetrennt werden, wobei ein flüssiges Gemisch enthaltend 30 - 70 Gew.-% Isocyanat, bevorzugt TDI, und 30 - 70 Gew.-% Rückstand erhalten wird. Dieses Gemisch wird anschließend in der Rückstandshydrolyse weiter aufgearbeitet. Bei der Hydrolyse des Rückstands handelt es sich um eine Umsetzung mit Wasser, die sogenannte Hydrolyse. Die Hydrolyse des Rückstandes wird im Allgemeinen durch Basen oder Säuren begünstigt. Die Hydrolyse kann dazu genutzt werden, den TDI-Destillationsrückstand zu denaturieren, wie in US-A-4,091,009 beschrieben. Eine weitere Möglichkeit ist die Rückgewinnung von TDA, welches dann im Isocyanatprozess unter Zugabe von Phosgen zu TDI umgesetzt werden kann. Derartige Verfahren werden beispielsweise in DE-A-29 42 678, JP-A-5 8201 751 und DE-A-19 62 598 beschrieben. Die Rückstandshydrolyse kann sowohl in Batchprozessen als auch kontinuierlich, z.B. in einem Röhrenreaktor, durchgeführt werden. Bevorzugte Temperaturen liegen im Bereich von 100°C bis 280°C und der Druck liegt bevorzugt zwischen 1 bar und 50 bar.

Die Hydrolyse des Destillationsrückstandes wird ohne Bitumen betrieben sowie mit schwefelarmen Chemikalien und ermöglicht deshalb eine deutliche Reduzierung der Schwefelbelastung im HCl-Abgas zur Chlorwasserstoffoxidation.

Daher wird in Schritt d) bevorzugt der Strom, der im Wesentlichen Isocyanat und nicht verdampfbaren Rückstand enthält, in einer Druckhydrolyse mit Wasser umgesetzt, wobei die primären Amine erhalten werden. Bevorzugt ist dieses Verfahren insbesondere für den Fall, dass das Isocyanat TDI ist.

Eine Kombination der beschriebenen Schritte, wie z.B. eine Rückstandshydrolyse mit einer anschließenden absorptiven Reinigung des HCl-Gases, ist durchaus geeignet um die Schwefelbelastung noch weiter zu verringern.

Grundsätzlich kann auf die Aufarbeitung des Gemisches aus Isocyanatprodukt und Destillationsrückstand in Schritt d) auch verzichtet werden und dieses kontinuierlich oder diskontinuierlich verbrannt werden. Das Verfahren ist technisch einfach und kann zur Nutzdampferzeugung eingesetzt werden, sofern eine dafür geeignete Anlage zur thermischen Verwertung in der Nähe der Isocyanat-Produktionsanlage existiert, um eine Entsorgung über eine Rohrleitungsanbindung zu gewährleisten.

Die Aufgabe der vorliegenden Erfindung zur Bereitstellung eines Chlorwasserstoffhaltigen Prozessgas einer Isocyanat Anlage für ein sich anschließendes Deacon oder Deacon-ähnliches Verfahren zur Oxidation des Chlorwasserstoffes mit Sauerstoff wurde zusammenfassend in Schritt d) dadurch gelöst, dass die Aufarbeitung des nicht verdampfbaren TDI-Destillationsrückstandes in Schritt d) durch Zusatz von schwefelarmen mit Gesamt-Schwefelgehalten kleiner 5-Gew.%, bevorzugt nahezu schwefelfreien Bitumen mit Gesamt-Schwefelgehalten von kleiner 1-Gew.% erreicht werden kann.

Weiterhin wird die Aufgabe der vorliegenden Erfindung gelöst, in dem die Aufarbeitung des nicht verdampfbaren TDI-Destillationsrückstandes in Knetertrocknern mit mehreren Wellen ohne Zusatz von Bitumen erfolgt kann.

Weiterhin kann die Aufgabe der vorliegenden Erfindung gelöst werden, in dem die Aufarbeitung des nicht verdampfbaren TDI-Destillationsrückstandes in Schritt d) hydrolytisch erfolgen kann. Bevorzugt werden dabei nahezu schwefelfreie Zuschlagsstoffe mit einem Gesamt-Schwefelgehalt kleiner 1 Gew.% eingesetzt.

Weiterhin kann die Aufgabe der vorliegenden Erfindung gelöst werden, in dem auf eine Aufarbeitung des nicht verdampfbaren TDI-Destillationsrückstandes in Schritt d) verzichtet wird, und dieser Rückstand z.B. einer thermischen Verwertung wie z.B. einer Verbrennung unterzogen wird.

Alternativ kann der HCl Prozessgasstrom aus Schritt b), der Gasstrom enthaltend Chlorwasserstoff, zunächst in einer Absorption, einer Adsorption (Aktivkohle, Adsorptionsmittel) oder einem Guard-Bed von Schwefel und Schwefelverbindungen gereinigt werden, bevor er in Schritt e) in die HCl-Oxidation gelangt.

In Schritt e) wird aus dem in Schritt b) erhaltenen Gasstrom enthaltend Chlorwasserstoff durch katalytische Oxidation Chlor hergestellt, wobei der Gehalt an Schwefel in elementarer oder gebundener Form in dem in der katalytischen Oxidation eingesetzte Gasstrom enthaltend Chlorwasserstoff insgesamt weniger als 100ppm, bevorzugt weniger als 50ppm, besonders bevorzugt weniger als 5ppm, ganz besonders bevorzugt weniger als 1ppm, insbesondere ganz besonders bevorzugt 0,001 bis 1 ppm, bezogen auf das Gewicht des Chlorwasserstoffstroms, beträgt. Somit ist es erforderlich, den in Schritt b) erhaltenen ersten Gasstrom enthaltend Chlorwasserstoff in der geforderten Reinheit bzgl. Schwefel zur Verfügung zu stellen. Dieses kann durch die beschriebenen Maßnahmen erreicht werden. Somit kann es ggf. erforderlich sein, die in Schritt b) erhaltenen Gasströme enthaltend Chlorwasserstoff vor ihrem Einsatz in der katalytischen Oxidation zu Chlor zu reinigen.

Die Reinigung des in den Schritten b) erhaltenen HCl-Gases von Schwefel in elementarer oder gebundener Form kann nicht über eine Wasserwäsche erfolgen, da dort die HCl absorbiert würde und somit zwangsläufig HCl-Verluste entstehen würden. Eine vollständige Absorption der HCl in Wasser zu Salzsäure, Reinigung der Salzsäure und anschließende Desorption der HCl aus der wässrigen Salzsäure ist energetisch sehr aufwändig und bietet ggf. eine unzureichende Reinigung. Somit ist die Aufreinigung schwierig.

Eine bevorzugte Reinigungsmethode der HCl sind Adsorptionsverfahren mit z.B. Aktivkohle oder verschiedenen keramischen Absorptionsmaterialien, wie Aluminiumoxid, Siliziumoxid, Zirkonoxid, etc. sowie gemischte Oxide. Bevorzugt werden regenerierbare Adsorptionsmaterialien verwendet. Die Adsorptionsmaterialien können mit Metallen oder Metallverbindungen imprägniert sein. Mögliche Verbindungen sind z.B. Kupferoxid, Lanthanoxid, Zinkoxid, Titanoxid, Zinktitanat, Eisenoxid, Kalziumoxid, Silikat und Aluminiumoxid, sowie Mischverbindungen. Es können auch mehrere hintereinander geschaltete Adsorptionsbetten eingesetzt werden, deren Inhalt differieren kann. Dieser Adsorptionsschritt kann in einem Festbett oder in einer Wirbelschicht, bevorzugt Festbett, durchgeführt werden, in einem Temperaturbereich von 0°C bis 600°C, bevorzugt von 20°C bis 400°C und einem Druckbereich der zwischen 1 bar und 50 bar liegt, bevorzugt im Bereich der im folgenden Schritt eingesetzten HCl-Oxidation. Die Regeneration des Adsorptionsbetts kann durch Abstellen des HCl-Stroms und anschließendes Einleiten eines inerten Gases, ggf. mit weiteren Bestandteilen wie Sauerstoff oder Wasserstoff, unter erhöhter Temperatur erfolgen.

Eine weitere, bevorzugte Möglichkeit besteht darin, ein Guard-Bed-Konzept zu integrieren. Hierbei wird der Chlorwasserstoff und ggf. weitere Gase wie z.B. Sauerstoff oder Chlor oder ein Gemisch aus mehreren Gasen über einen Katalysator geführt. Dabei werden die Schwefelkomponenten über eine chemische Reaktion (z.B. Oxidation und/oder Komplexbildung) abgeschieden. Bei diesem Konzept kann man eine gleichzeitige Entfernung weiterer Bestandteile über eine chemische Reaktion gewährleisten. So ist eine Oxidation von Kohlenmonoxid, ein weiteres mögliches Katalysatorgift, sowie Kohlenwasserstoffe, substituiert wie unsubstituiert, in Anwesenheit von Sauerstoff möglich. Diese Art der Auslegung der Erfindung kann sowohl in einem Festbettreaktor als auch in einem Wirbelschichtreaktor durchgeführt werden. Eine solche Aufreinigung ist beispielsweise in JP2005-177614 beschrieben.

Alternativ kann der Gasstrom aus den Rückstandspfannen oder Knetertrockner über eine Adsorption bzw. Guard-Bed direkt von Schwefel gereinigt werden, bevor er wieder in den Phosgenierungsschritt eingespeist wird. Dies hat den Vorteil, dass man deutlich geringere Gasmengen aufreinigen muss.

In Schritt d) wird das aus dem in Schritt c) erhaltenen Gemisch enthaltend Isocyanat und nicht verdampfbarem Rückstand ein Gasstrom enthaltend Isocyanat und ggf. vorhandenes Lösungsmittel sowie ggf. vorhandene Spuren an Schwefelverbindungen abgetrennt. In einer bevorzugten Ausführungsform erfolgt die Abtrennung von ggf. noch vorhandenem Chlorwasserstoff aus dem Gemisch enthaltend Isocyanat und nicht verdampfbarem Rückstand ebenfalls in Schritt d). Diese Ausführungsform ist besonders bevorzugt, wenn in Schritt d) Knetertrockner oder Rückstandspfannen eingesetzt werden. Bevorzugt wird der so erhaltene Gasstrom enthaltend Isocyanat und ggf. Lösungsmittel anschließend in einer Absorption oder in einem Guard Bed von Schwefel und Schwefelverbindungen gereinigt.

Allen genannten Methoden ist gemeinsam, dass sie zu einer Verringerung des Schwefelanteils in dem Prozessgas einer Isocyanatanlage zu einem Deacon-Prozess führen. In jedem Fall ist die Entfernung von Schwefel aus dem Isocyanatprozessgas ein zusätzlicher, oft aufwändiger Reinigungsschritt in dem Deacon-Prozess bevor das Gas in Kontakt mit dem Katalysator gerät. Vergleichsweise einfach ist die Ausführungsform, in der in Schritt d) der Rückstand durch Hydrolyse aufgearbeitet wird.

Es ist aber auch vorteilhaft, die Reinheit des HCl-Gases der Isocyanatanlage hinsichtlich des Gehalts an Schwefelkomponenten dadurch zu beeinflussen, dass Schwefelquellen im gesamten Isocyanat-Verfahren selbst reduziert werden. Bevorzugt wird daher der Schwefelgehalt im CO-haltigen Eingangsstrom in die Phosgenherstellung in Schritt a) reduziert um so eine Kontamination des HCl Abgasstroms zu vermeiden.

Eine weitere Möglichkeit einen niedrigen Gehalt an Schwefel im Phosgen einzustellen, ist beispielsweise die Verwendung qualitativ höherwertiger Edukte bei der Phosgenherstellung, die bereits einen entsprechend niedrigen Gehalt an Schwefel aufweisen. Insbesondere bietet sich hierbei die Verwendung von CO mit einem entsprechend niedrigen Gehalt an Schwefel an, bevorzugt unterhalb von 100ppm, bevorzugter unterhalb von 10ppm besonders bevorzugt unterhalb von 1ppm. Verfahren zur Herstellung von CO mit einem niedrigen Gehalt an Schwefel sind in der Fachwelt bekannt. So kann beispielsweise CO eingesetzt werden, das durch Kohlevergasung, Dampf-Reforming, CO₂-Reforming, partielle Oxidation von Kohlenwasserstoffen oder anderen Verfahren gewonnen wurde. CO kann ebenfalls durch Abtrennung aus CO-haltigen Gasgemischen gewonnen werden. Derartige Verfahren zur Herstellung bzw. Gewinnung von CO werden z. B. in Chemische Technik (Hrsg.: Dittmeyer, Keim, Kreysa, Oberholz), 5. Aufl., Bd. 4, Seite 981-1007 beschrieben.

Der erforderliche niedrige Schwefelgehalt im CO wird erreicht, indem für die CO-Herstellung bzw. -Gewinnung Rohstoffe eingesetzt werden, die praktisch schwefelfrei sind oder einen ausreichend niedrigen Schwefelgehalt aufweisen. Dabei ist es unerheblich, wie die Schwefelfreiheit bzw. der ausreichend niedrige Schwefelgehalt der für die CO-Herstellung bzw. -Gewinnung eingesetzten Rohstoffe erreicht wird.

Eine andere Möglichkeit, den erforderlichen niedrigen Schwefelgehalt im CO zu erreichen, besteht in der Entfernung von Schwefel (in elementarer oder gebundener Form vorliegend) aus dem einzusetzenden CO. In der Literatur sind zahlreiche Verfahren beschrieben, die diesem Zweck dienen. Beispielhaft sei die Entfernung von schwefelhaltigen Verunreinigungen wie H₂S gemäß US-A1-2005/0025693 erwähnt, dadurch, dass der COhaltige Gasstrom mit Aktivkohle, welche mit einem Metalloxid imprägniert wurde, in Kontakt gebracht wird. Eine andere Möglichkeit ist z.B. gemäß DE-A1-103 01 434 die Konvertierung anorganischer und organischer Schwefelverbindungen in Gegenwart von Wasserdampf an einem Aluminiumoxid-Kontakt bei erhöhter Temperatur und Überleiten des erhaltenen Gasgemisches über eine Eisenhydroxidhaltige Masse in Gegenwart von Wasserdampf und definierter Mengen an Sauerstoff. DE-A1-103 01 434 zitiert noch weitere Möglichkeiten, CO von schwefelhaltigen Verunreinigungen zu befreien.

Das so erhältliche, weitgehend schwefelfreie CO kann anschließend im Rahmen üblicher und bekannter Prozesse wie sie z.B. in Ullmanns Enzyklopädie der industriellen Chemie, 3. Aufl., Bd. 13, Seite 494-500 beschrieben sind, zu Phosgen umgesetzt werden.

Ein weiterer Weg, Phosgen mit einem niedrigen Gehalt an elementarem oder gebundenem Schwefel zu erhalten, ist die Abtrennung von elementarem oder gebundenem Schwefel aus dem Phosgen selbst. Auch hier können im Prinzip wieder alle gängigen Trennverfahren eingesetzt werden, beispielsweise Destillation, Adsorption und dergleichen. Für das erfindungsgemäße Verfahren ist letztlich ausschließlich die Einhaltung der oben genannten Obergrenze für die Konzentration an elementarem oder gebundenem Schwefel entscheidend.

Bevorzugt enthält das in Schritt a) erhaltene Phosgen einen Gehalt an Schwefel in elementarer oder gebundener Form von weniger als 100 ppm.

Das erfindungsgemäße Verfahren zur Herstellung von Chlor durch katalytische Oxidation von Chlorwasserstoff, bei dem der eingesetzte Chlorwasserstoffstrom einen Gehalt an Schwefel in elementarer oder gebundener Form von weniger als 100ppm, bevorzugt weniger als 50ppm, besonders bevorzugt weniger als 5ppm, ganz besonders bevorzugt weniger als 1ppm, bezogen auf das Gewicht des Chlorwasserstoffstroms aufweist, ermöglicht es, z.B. bei der Kopplung mit einem Isocyanat-Prozess erheblich verlängerte Standzeiten des Deacon Katalysators zu erreichen, sowie im Fall von reversiblen oder teilreversiblen Vergiftungsprozessen verlängerte Deaktivierungszeiten und damit längere Lebensdauer des Katalysators zu erreichen.

Die katalytische Chlorwasserstoff-Oxidation in dem erfindungsgemäßen Verfahren kann adiabatisch oder isotherm oder annähernd isotherm, diskontinuierlich, bevorzugt aber kontinuierlich als Fließ- oder Festbettverfahren, bevorzugt als Festbettverfahren, besonders bevorzugt in Rohrbündelreaktoren an Heterogenkatalysatoren bei einer Reaktortemperatur von 180 bis 500°C, bevorzugt 200 bis 450°C, besonders bevorzugt 220 bis 400°C und einem Druck von 1 bis 25 bar (1000 bis 25000 hPa), bevorzugt 1,2 bis 20 bar, besonders bevorzugt 1,5 bis 17 bar und insbesondere 2,0 bis 15 bar durchgeführt werden.

Übliche Reaktionsapparate, in denen die katalytische Chlorwasserstoff-Oxidation durchgeführt wird, sind Festbett- oder Wirbelbettreaktoren. Die katalytische Chlorwasserstoff-Oxidation kann bevorzugt auch mehrstufig durchgeführt werden.

Bei der isothermen oder annähernd isothermen sowie adiabaten Fahrweise können auch mehrere, bevorzugt 2 bis 10, besonders bevorzugt 2 bis 6, ganz besonders bevorzugt 2 bis 5, insbesondere ganz besonders bevorzugt 2, 3 oder 4, in Reihe geschaltete Reaktoren mit zusätzlicher Zwischenkühlung eingesetzt werden. Der Chlorwasserstoff kann entweder vollständig zusammen mit dem Sauerstoff vor dem ersten Reaktor oder über die verschiedenen Reaktoren verteilt zugegeben werden. Diese Reihenschaltung einzelner Reaktoren kann auch in einem Apparat zusammengeführt werden.

Eine weitere bevorzugte Ausführungsform einer für das Verfahren geeigneten Vorrichtung besteht darin, dass man eine strukturierte Katalysatorschüttung einsetzt, bei der die Katalysatoraktivität in Strömungsrichtung ansteigt. Eine solche Strukturierung der Katalysatorschüttung kann durch unterschiedliche Tränkung der Katalysatorträger mit Aktivmasse oder durch unterschiedliche Verdünnung des Katalysators mit einem Inertmaterial erfolgen. Als Inertmaterial können beispielsweise Ringe, Zylinder oder Kugeln aus Titandioxid, Zirkondioxid oder deren Gemischen, Aluminiumoxid, Steatit, Keramik, Glas, Graphit, Edelstahl oder Nickellegierungen eingesetzt werden. Beim bevorzugten Einsatz von Katalysatorformkörpern sollte das Inertmaterial bevorzugt ähnliche äußere Abmessungen haben.

Als Katalysatorformkörper eignen sich Formkörper mit beliebigen Formen, bevorzugt sind Tabletten, Ringe, Zylinder, Sterne, Wagenräder oder Kugeln, besonders bevorzugt sind Ringe, Zylinder oder Stemstränge als Form.

Als Heterogenkatalysatoren eignen sich insbesondere Rutheniumverbindungen oder Kupferverbindungen auf Trägermaterialen, die auch dotiert sein können, bevorzugt sind gegebenenfalls dotierte Rutheniumkatalysatoren. Als Trägermaterialen eignen sich beispielsweise Siliziumdioxid, Graphit, Titandioxid mit Rutil- oder Anatas-Struktur, Zirkondioxid, Aluminiumoxid oder deren Gemische, bevorzugt Titandioxid, Zirkondioxid, Aluminiumoxid oder deren Gemische, besonders bevorzugt γ- oder δ-Aluminiumoxid oder deren Gemische.

Die Kupfer- bzw. die Rutheniumträgerkatalysatoren können beispielsweise durch Tränkung des Trägermaterials mit wässrigen Lösungen von CuCl₂ bzw. RuCl₃ und gegebenenfalls eines Promotors zur Dotierung, bevorzugt in Form ihrer Chloride, erhalten werden. Die Formgebung des Katalysators kann nach oder bevorzugt vor der Tränkung des Trägermaterials erfolgen.

Zur Dotierung der Katalysatoren eignen sich als Promotoren Alkalimetalle wie Lithium, Natrium, Kalium, Rubidium und Cäsium, bevorzugt Lithium, Natrium und Kalium, besonders bevorzugt Kalium, Erdalkalimetalle wie Magnesium, Calcium, Strontium und Barium, bevorzugt Magnesium und Calcium, besonders bevorzugt Magnesium, Seltenerdmetalle wie Scandium, Yttrium, Lanthan, Cer, Praseodym und Neodym, bevorzugt Scandium, Yttrium, Lanthan und Cer, besonders bevorzugt Lanthan und Cer, oder deren Gemische.

Die Formkörper können anschließend bei einer Temperatur von 100 bis 1000°C, bevorzugt 100 bis 500°C beispielsweise unter einer Stickstoff-, Argon- oder Luftatmosphäre getrocknet und gegebenenfalls calciniert werden. Bevorzugt werden die Formkörper zunächst bei 100 bis 150°C getrocknet und anschließend bei 200 bis 500°C calciniert.

Der Umsatz an Chlorwasserstoff im einfachen Durchgang kann bevorzugt auf 15 bis 90 %, bevorzugt 40 bis 85% begrenzt werden. Nicht umgesetzter Chlorwasserstoff kann nach Abtrennung teilweise oder vollständig in die katalytische Chlorwasserstoff-Oxidation zurückgeführt werden.

Die Reaktionswärme der katalytischen Chlorwasserstoff-Oxidation kann in vorteilhafter Weise zur Erzeugung von Hochdruck-Wasserdampf genutzt werden. Dieser kann z.B. zum Betrieb eines Phosgenierungsreaktors oder von Destillationskolonnen, insbesondere von Isocyanat-Destillationskolonnen genutzt werden.

Das nach dem erfindungsgemäßen Verfahren erhaltene Chlor kann anschließend zumindest zum Teil nach dem aus dem Stand der Technik bekannten Verfahren mit Kohlenmonoxid zu Phosgen umgesetzt werden, welches für die Herstellung von Isocyanaten aus dem korrespondierenden Aminen eingesetzt werden kann. Der bei der Phosgenierung der Amine wiederum entstehende Chlorwasserstoff kann anschließend nach den beschriebenen Verfahren zu Chlor umgesetzt werden.

Durch das erfindungsgemäße Verfahren wird der Schwefel-Gehalt im HCl-Strom deutlich verringert, wodurch die Standzeit des Deacon-Katalysators in der nächsten Stufe verlängert wird und eine mögliche Abscheidung der Schwefelkomponente (katalysatorabhängig) führt langsamer zu einer verringerten Deaktivierung des Katalystors.

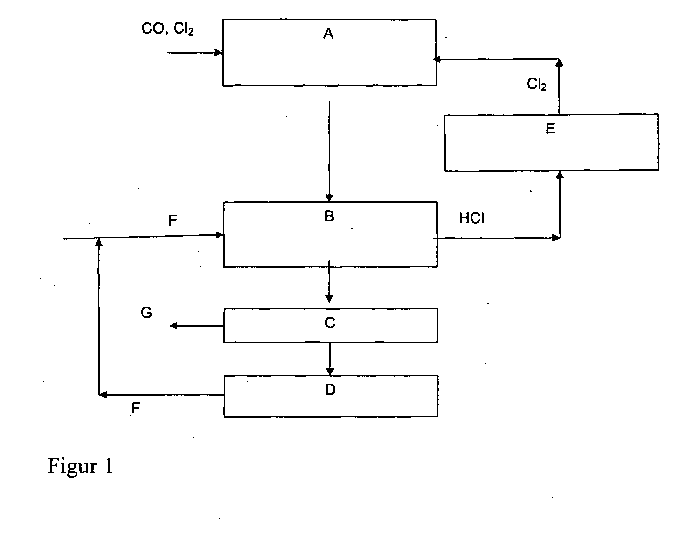

Figur 1 zeigt beispielhaft eine bevorzugte Ausführungsform des erfindungsgemäßen Verfahrens zur Herstellung von Isocyanaten, in dem die folgenden Zeichen die folgende Bedeutung haben:
A: Phosgensynthese (Schritt a))
B: Isocyanatsynthese (Schritt b))
C: Destillation (Schritt c))
D: Rückstandshydrolyse (Schritt d))
E: katalytische HCl-Oxidation
F: Amin
G: Isocyanat:

Das in E hergestellte Chlor wird in die Phosgensynthese in A zurückgeführt. Das in D zurückgewonnene Amin F wird in die Phosgenierung in B zurückgeführt.

### Beispiele

### Beispiel 1:

10 g Rutheniumchlorid-n-Hydrat werden in 34 ml Wasser gelöst und 200 g Träger (SnO₂/Al₂O₃ (85:15 m/m); 1,5 mm) dazugegeben und solange durchmischt bis die Lösung vom Träger aufgenommen worden ist. Der so imprägnierte Träger wird 1 h stehen gelassen. Der feuchte Feststoff wird abschließend ungewaschen im Muffelofen für 4 h bei 60°C und 16 h bei 250°C getrocknet.

2 g des getrockneten Katalysators werden bei 300°C bei einem Gasfluss von 8 L/h (STP) Sauerstoff, 2 L/h (STP) Chlorwasserstoff und 10 L/h (STP) Stickstoff durchströmt. Die Menge an gebildetem Chlor wird über Einleiten in eine 16%-ige Kaliumiodidlösung und Titration des entstandenen Iods mit Thiosulfat bestimmt. Es ergibt sich eine im zeitlichen Verlauf konstante Raumzeitausbeute von 0,48 kg Chlor /(kg Kat. h).

In weiteren Versuchen werden anhand von Perfusorpumpen bestimmte Mengen an Schwefelkomponenten zugegeben unter sonst gleich gebliebenen Bedingungen. Die Mengenangaben für Schwefelverbindungen in ppm beziehen sich auf den gesamten Gasstrom. Der Abfall der Raumzeitausbeite (Aktivität) des Katalysators nach 10 Stunden Vergiftungszeit mit SO₂ und COS ist in Tabelle 1 angegeben.

**Tabelle 1**

| Schwefelkomponente | Menge Schwefelkomponente (ppm) | Aktivitätsabfall in 10h |
|---|---|---|
| SO₂ | 525 | 69% |
| SO₂ | 158 | 55% |
| SO₂ | 53 | 11% |
| COS | 53 | 32% |
| COS | 11 | 20% |

### Beispiel 2:

Phosgen erzeugt durch Umsetzung von Kohlenmonoxid mit Chlor an A-Kohle werden bei Temperaturen größer 300 °C in der Gasphase mit 2.5 Tonnen gasförmigen TDA zu rohem TDI umgesetzt. Ein HCl Teilstrom wird nach Reinigung über eine Tiefkältefalle katalytisch zu Chlor oxidiert. Das rohe TDI wird destillativ gereinigt, wobei ein TDI-Rückstandsgemisch mit ca. 20 Gew.-% TDI-Destillationsrückstand erhalten wird, dass über einen Verdampfer auf ca. 50 Gew.% aufkonzentriert wird. Aus diesem Gemisch wird unter Zusatz von Bitumen mit einem Schwefelgehalt < 5 Gew.% weiteres TDI gewonnen.

### Beispiel 3:

Eine Phosgenlösung in ODB (Ortho-Dichlorbenzol), die durch Lösen von Phosgen, dass aus Chlor und Kohlenmonoxid an A-Kohle unter gleichzeitiger Erzeugung von Dampf hergestellt wird, wird mit einer TDA Lösung in ODB umgesetzt und liefert rohes TDI. Dieses TDI wird destillativ gereinigt und man erhält reines TDI und ein Gemisch bestehend aus TDI mit ca. 10 Gew.-% TDI-Destillationsrückstand. Dieses Rückstandsgemisch wird auf 50 Gew.-% TDI-Destillationsrückstand aufkonzentriert und kontinuierlich in einem Knetertrockner mit Bitumen versetzt, wobei weiteres TDI gewonnen wird. Die Vakuumablüfte des Knetertrockners werden mit ODB gewaschen und die erhaltene Waschflüssigkeit wird in der Phosgenierung wieder verwendet. Der HCl Strom wird über eine bei - 35 °C betriebene Tiefkältefalle und anschließend über einen mit Aktiv-Kohle gefüllten Absorber gereinigt und mit einem Schwefelgehalt von kleiner 1 ppm katalytisch zu Chlor oxidiert, wobei 50 kg/h Chlor erhalten werden. Das verwendete Lösemittel hat einen Schwefelgehalt von 4 ppm.

## Patentansprüche

1. Verfahren zur Herstellung von Isocyanaten durch Phosgenierung der entsprechenden Amine, in dem
a) Kohlenmonoxid mit Chlor zu Phosgen umgesetzt wird, und
b) die Amine mit Phosgen zu den Isocyanaten umgesetzt werden, wobei ein rohes Isocyanatgemisch und ein Gasstrom enthaltend Chlorwasserstoff erhalten wird, und
c) das rohe Isocyanatgemisch destillativ gereinigt wird, wobei das Isocyanat und ein Gemisch enthaltend Isocyanat und nicht verdampfbaren Rückstand in Gehalten von 10 bis 70 Gew.-% nicht verdampfbarer Isocyanat-Rückstand, bezogen auf das Gewicht des Gemisches erhalten wird, und
d) das in dem Schritt c) erhaltene Gemisch aus Isocyanat und nicht verdampfbarem Rückstand in Gehalten von 10 bis 70 Gew.-% einem weiteren Aufarbeitungsschritt zugeführt wird, bei dem Isocyanat durch Verdampfung in einem Knetertrockner oder in Rückstandspfannen zurück gewonnen wird oder das Amin durch Hydrolyse des Rückstands zurück gewonnen wird und, das zurückgewonnene Isocyanat oder das zurückgewonnene Amin zumindest teilweise in die Umsetzung in Schritt b) zurückgeführt wird, und
e) aus dem in Schritt b) erhaltenen Gasstrom enthaltend Chlorwasserstoff durch katalytische Oxidation Chlor hergestellt wird, wobei der Gehalt an Schwefel in elementarer oder gebundener Form in dem in der katalytischen Oxidation eingesetzte Gasstrom enthaltend Chlorwasserstoff insgesamt weniger als 100ppm, bevorzugt weniger als 50ppm, besonders bevorzugt weniger als 5ppm, ganz besonders bevorzugt weniger als 1ppm, bezogen auf das Gewicht des eingesetzten Gasstroms, beträgt, und
f) das in Schritt e) hergestellte Chlor zumindest in Teilen in die Herstellung von Phosgen in Schritt a) zurückgeführt wird.

2. Verfahren nach Anspruch 1, bei dem in Schritt d) die Abtrennung des Isocyanats vom Rückstand in einem Knetertrockner oder in Rückstandspfannen erfolgt, wobei Bitumen mit Schwefelgehalten von < 5 Gew.-% Schwefel, bezogen auf das Gesamtgewicht des Bitumens, eingesetzt wird.

3. Verfahren nach Anspruch 1 oder 2, bei dem in Schritt d) die Abtrennung des Isocyanates vom Rückstand in einem Knetertrockner oder Rückstandspfannen erfolgt, und der Gasstrom enthaltend Chlorwasserstoff der in Schritt e) zur HCl-Oxidation gelangt in einer Absorption von Schwefel gereinigt wird.

4. Verfahren nach Anspruch 1 oder 2, bei dem in Schritt d) die Abtrennung des Isocyanates vom Rückstand in einem Knetertrockner oder Rückstandspfannen erfolgt, und der Gasstrom enthaltend Chlorwasserstoff der in Schritt e) zur HCl-Oxidation gelangt in einem Guard-Bed von Schwefel gereinigt wird.

5. Verfahren nach Anspruch 1 oder 2, bei dem in Schritt d) die Abtrennung des Isocyanates vom Rückstand in einem Knetertrockner oder Rückstandspfannen erfolgt, und der dabei erhaltene Gasstrom enthaltend Isocyanat anschließend in einer Absorption von Schwefel gereinigt wird.

6. Verfahren nach Anspruch 1 oder 2, bei dem in Schritt d) die Abtrennung des Isocyanates vom Rückstand in einem Knetertrockner oder Rückstandspfannen erfolgt, und der dabei erhaltene Gasstrom enthaltend Isocyanat anschließend in einem Guard-Bed von Schwefel gereinigt wird.

7. Verfahren nach Anspruch 1, bei dem in Schritt a) CO und / oder Phosgen über ein Adsorptionsmittel von Schwefel befreit wird.

8. Verfahren nach Anspruch 1, bei dem in Schritt a) ein Phosgen erzeugt wird, das einen Gehalt an Schwefel in elementarer oder gebundener Form von weniger als 100 ppm aufweist.

9. Verfahren nach Anspruch 1, bei dem die Umsetzung der Amine mit Phosgen in Schritt b) in der Gasphase durchgeführt wird.

## Claims

1. Process for preparing isocyanates by phosgenating the corresponding amines, in which
a) carbon monoxide is reacted with chlorine to give phosgene, and
b) the amines are reacted with phosgene to give the isocyanates, which affords a crude isocyanate mixture and a gas stream comprising hydrogen chloride, and
c) the crude isocyanate mixture is purified by distillation to obtain the isocyanate and a mixture comprising isocyanate and unvaporizable residue in contents of 10% to 70% by weight of unvaporizable isocyanate residue, based on the weight of the mixture, and
d) the mixture of isocyanate and unvaporizable residue in contents of 10% to 70% by weight obtained in step c) is sent to a further workup step in which isocyanate is recovered by vaporization in a kneader-dryer or in residue boilers, or the amine is recovered by hydrolysis of the residue and the recovered isocyanate or the recovered amine is at least partly recycled into the reaction in step b), and
e) chlorine is prepared by catalytic oxidation from the gas stream which comprises hydrogen chloride and is obtained in step b), the total sulphur content in elemental or bound form in the gas stream which comprises hydrogen chloride and is obtained in the catalytic oxidation being less than 100 ppm, preferably less than 50 ppm, more preferably less than 5 ppm, most preferably less than 1 ppm, based on the weight of the gas stream used, and
f) the chlorine prepared in step e) is recycled at least in portions into the preparation of phosgene in step a).

2. Process according to Claim 1, in which the isocyanate is removed from the residue in step d) in a kneader-dryer or in residue boilers using bitumen with sulphur contents of < 5% by weight of sulphur, based on the total weight of the bitumen.

3. Process according to Claim 1 or 2, in which the isocyanate is removed from the residue in step d) in a kneader-dryer or residue boilers, and the gas stream comprising hydrogen chloride which is passed to the HCl oxidation in step e) is purified to remove sulphur in an absorption.

4. Process according to Claim 1 or 2, in which the isocyanate is removed from the residue in step d) in a kneader-dryer or residue boilers, and the gas stream comprising hydrogen chloride which is passed to the HCl oxidation in step e) is purified to remove sulphur in a guard bed.

5. Process according to Claim 1 or 2, in which the isocyanate is removed from the residue in step d) in a kneader-dryer or residue boilers, and the resulting gas stream comprising isocyanate is subsequently purified to remove sulphur in an absorption.

6. Process according to Claim 1 or 2, in which the isocyanate is removed from the residue in step d) in a kneader-dryer or residue boilers, and the resulting gas stream comprising isocyanate is subsequently purified to remove sulphur in a guard bed.

7. Process according to Claim 1, in which, in step a), CO and/or phosgene is freed of sulphur by means of an adsorbent.

8. Process according to Claim 1, in which, in step a), phosgene having a sulphur content in elemental or bound form of less than 100 ppm is obtained.

9. Process according to Claim 1, in which the reaction of the amines with phosgene in step b) is performed in the gas phase.

## Revendications

1. Procédé de fabrication d'isocyanates par phosgénation des amines correspondantes, dans lequel
a) du monoxyde de carbone est transformé en phosgène avec du chlore, et
b) les amines sont transformées en isocyanates avec du phosgène, un mélange d'isocyanates brut et un courant gazeux contenant du chlorure d'hydrogène étant obtenus, et
c) le mélange d'isocyanates brut est purifié par distillation, l'isocyanate et un mélange contenant de l'isocyanate et un résidu non vaporisable en teneurs de 10 à 70 % en poids de résidu d'isocyanate non vaporisable, par rapport au poids du mélange, étant obtenus, et
d) le mélange de l'isocyanate et du résidu non vaporisable en teneurs de 10 à 70 % en poids obtenu à l'étape c) est introduit dans une étape de traitement supplémentaire, lors de laquelle l'isocyanate est récupéré par évaporation dans un séchoir-malaxeur ou dans des poêles à résidu, ou l'amine est récupérée par hydrolyse du résidu, et l'isocyanate récupéré ou l'amine récupérée est recyclé au moins en partie dans la réaction de l'étape b), et
e) du chlore est fabriqué par oxydation catalytique à partir du courant gazeux contenant du chlorure d'hydrogène obtenu à l'étape b), la teneur en soufre sous forme élémentaire ou reliée dans le courant gazeux contenant du chlorure d'hydrogène utilisé dans l'oxydation catalytique étant au total inférieure à 100 ppm, de préférence inférieure à 50 ppm, de manière particulièrement préférée inférieure à 5 ppm, de manière tout particulièrement préférée inférieure à 1 ppm, par rapport au poids du courant gazeux utilisé, et
f) le chlore fabriqué à l'étape e) est recyclé au moins en parties dans la fabrication du phosgène à l'étape a).

2. Procédé selon la revendication 1, dans lequel la séparation de l'isocyanate du résidu à l'étape d) a lieu dans un séchoir-malaxeur ou dans des poêles à résidu, un bitume ayant des teneurs en soufre < 5 % en poids de soufre, par rapport au poids total du bitume, étant utilisé.

3. Procédé selon la revendication 1 ou 2, dans lequel la séparation de l'isocyanate du résidu à l'étape d) a lieu dans un séchoir-malaxeur ou dans des poêles à résidu, et le courant gazeux contenant du chlorure d'hydrogène qui atteint l'oxydation d'HCl à l'étape e) est purifié du soufre dans une absorption.

4. Procédé selon la revendication 1 ou 2, dans lequel la séparation de l'isocyanate du résidu à l'étape d) a lieu dans un séchoir-malaxeur ou dans des poêles à résidu, et le courant gazeux contenant du chlorure d'hydrogène qui atteint l'oxydation d'HCl à l'étape e) est purifié du soufre dans un lit de garde.

5. Procédé selon la revendication 1 ou 2, dans lequel la séparation de l'isocyanate du résidu à l'étape d) a lieu dans un séchoir-malaxeur ou dans des poêles à résidu, et le courant gazeux contenant de l'isocyanate obtenu est ensuite purifié du soufre dans une absorption.

6. Procédé selon la revendication 1 ou 2, dans lequel la séparation de l'isocyanate du résidu à l'étape d) a lieu dans un séchoir-malaxeur ou dans des poêles à résidu, et le courant gazeux contenant de l'isocyanate obtenu est ensuite purifié du soufre dans un lit de garde.

7. Procédé selon la revendication 1, dans lequel le CO et/ou le phosgène sont débarrassés du soufre par un agent d'adsorption lors de l'étape a).

8. Procédé selon la revendication 1, dans lequel un phosgène qui présente une teneur en soufre sous forme élémentaire ou reliée inférieure à 100 ppm est formé à l'étape a).

9. Procédé selon la revendication 1, dans lequel la réaction des amines avec le phosgène à l'étape b) est réalisée en phase gazeuse.
